# EUROPEAN PATENT APPLICATION

(11) **EP 4 831 641 A1**
(43) Date of publication of application: **28.10.2026**
(21) Application number: 24906219.1
(22) Date of filing: 12.12.2024
(51) Int. Cl.: G01N 35/04

(54) **CONTAINER MANIPULATION DEVICE AND MEDICAL DETECTION ANALYZER COMPRISING SAME**

(30) Priority: 21.12.2023 CN 202311770701
(71) Applicant: Beckman Coulter Laboratory Systems (Suzhou) Co. Ltd., Suzhou, Jiangsu 215126 (CN)
(72) Inventor: ZHAO, Liang, Suzhou, Jiangsu 215126 (CN); ZUO, Lidang, Suzhou, Jiangsu 215126 (CN)
(74) Representative: Maiwald GmbH
(86) International application number: PCT/CN2024/138692
(87) International publication number: WO 2025/130736

(57) **Abstract**

A container manipulation device for a medical detection analyzer, and the medical detection analyzer comprising the container manipulation device. The container manipulation device comprises: a clamping member which comprises an elastic clamping portion for clamping a container; an ejection member which is slidably arranged in the clamping member; a biasing member which is configured to apply force to the ejection member, thus enabling the ejection member to push the container out of the elastic clamping portion; a locking mechanism which is configured such that, when the container is clamped in place by the elastic clamping portion, the locking mechanism maintains the ejection member in a standby state where the force applied by the biasing member will not be transmitted to the container; and an unlocking mechanism which is configured to release the standby state of the ejection member so as to push the container out of the clamping member under the action of the biasing member. The container manipulation device and the medical detection analyzer can simplify the structure, reduce the cost, improve the reliability and stability, etc.

## Description

The present application claims the priority to the Chinese Patent Application No. 202311770701.9, titled "CONTAINER MANIPULATION DEVICE AND MEDICAL DETECTION ANALYZER COMPRISING SAME", filed with the China National Intellectual Property Administration on December 21, 2023, which is incorporated herein by reference in its entirety.

### FIELD

The present disclosure relates to a container manipulation device for clamping and releasing containers (e.g., reaction cuvettes) and a medical detection analyzer (e.g., an immunoassay analyzer) including the container manipulation device.

### BACKGROUND

The content of this section merely provides background information related to the present disclosure and does not necessarily constitute prior art.

A medical detection analyzer may be used to detect and analyze certain biochemical substances in body fluids (for example, blood), and provide informational basis for clinical immunity, diagnosis, treatment, prognosis, and health status of diseases. A medical detection analyzer generally includes a container manipulation device, which is used to clamp, transport, mix, or place containers (which may also be referred to as "reaction cuvettes" or "test tubes") containing detection substances or reagents therein, so as to position the containers at appropriate locations.

A conventional container manipulation device includes a clamping member, an ejection member, and a pneumatic system. The clamping member is used to pick up and clamp a container. The pneumatic system applies pressurized gas onto the ejection member so as to push the ejection member and thereby push the container, such that the container is ultimately released from the clamping member and placed at an appropriate location.

### SUMMARY

### Technical Problem

For the above-described conventional container manipulation device, the pneumatic system includes various pipelines, valves, sensors, regulating devices, or the like, resulting in a complex structure, large volume, and high cost. Therefore, the container manipulation device and the medical detection analyzer including the pneumatic system also have complex structures and operations, large volumes, and high costs. In addition, when the container is ejected under force, it is easy to cause the fluid inside the container to splash out, thereby causing problems such as contamination. The pneumatic system also suffers from issues of air leakage and pressure regulation. This reduces the stability and reliability of the pneumatic system.

In view of at least one of the above-described problems of the pneumatic system, a container manipulation solution has been proposed, which utilizes a novel method of applying force and ejecting the container to replace the pneumatic system, thereby simplifying the structure, making the structure more compact, reducing costs, and/or improving stability and reliability.

### Technical Solution

According to one aspect of the present application, a container manipulation device for a medical detection analyzer is provided. The container manipulation device includes: a clamping member including an elastic clamping portion for clamping a container; an ejection member disposed in the clamping member in a slidable manner; a biasing member configured to apply a force to the ejection member so that the ejection member pushes the container out from the elastic clamping portion; a locking mechanism configured such that when the container is clamped in place by the elastic clamping portion, the locking mechanism holds the ejection member in a standby state in which the force applied by the biasing member will not be transmitted to the container; and an unlocking mechanism configured to release the standby state of the ejection member so that the container is pushed out from the clamping member under the action of the biasing member.

In some embodiments according to the present application, the container manipulation device further includes a housing, and a bearing is provided between the housing and the clamping member. The housing is configured to be coupled to a linear drive mechanism and, under the drive of the linear drive mechanism, to move linearly together with the clamping member. The clamping member is configured to be coupled to a rotary drive mechanism and, under the drive of the rotary drive mechanism, to rotate relative to the housing.

In some embodiments according to the present application, the container manipulation device includes a cam disposed between the biasing member and the ejection member. The locking mechanism includes a first engagement portion provided on the cam and a second engagement portion provided on the clamping member, and the first engagement portion is configured to be engaged with the second engagement portion to place the ejection member in the standby state. The unlocking mechanism is provided on the ejection member.

In some embodiments according to the present application, the cam includes a cylindrical cam body and cam rib portions provided on an outer peripheral surface of the cam body. The first engagement portion is provided on the cam rib portions. The clamping member includes a cylindrical clamping member body and clamping member rib portions provided on an inner peripheral surface of the clamping member body. The second engagement portion is provided on the clamping member rib portions.

In some embodiments according to the present application, the cam is configured to be rotatable between a first circumferential position and a second circumferential position. When the cam is in the first circumferential position, each of the clamping member rib portions is located between adjacent cam rib portions in the circumferential direction so as to enable relative axial sliding between the clamping member and the cam. When the cam is in the second circumferential position, the first engagement portion is engaged with the second engagement portion.

In some embodiments according to the present application, the clamping member rib portions extend in an axial direction; and/or the cam rib portions extend in the axial direction.

In some embodiments according to the present application, the first engagement portion is provided at an end portion of the cam rib portions, and the second engagement portion is provided at an end portion of the clamping member rib portions.

In some embodiments according to the present application, each of the first engagement portion, the second engagement portion, and the unlocking mechanism has an inclined surface. The container manipulation device is configured such that, under the interaction of the inclined surface of the first engagement portion with the inclined surface of the second engagement portion, and under the interaction of the inclined surface of the first engagement portion with the inclined surface of the unlocking mechanism, the cam can be rotated between the first circumferential position and the second circumferential position.

In some embodiments according to the present application, the second engagement portion has a stop portion configured to allow the first engagement portion to rest against the clamping member.

In some embodiments according to the present application, the ejection member includes a cylindrical ejection member body and ejection member rib portions provided on an outer peripheral surface of the ejection member body. The ejection member rib portions are located between adjacent clamping member rib portions in the circumferential direction, so that the ejection member can slide axially relative to the clamping member but cannot rotate. The unlocking mechanism is provided on an end face of the ejection member body facing the cam.

In some embodiments according to the present application, the ejection member rib portions extend in an axial direction.

In some embodiments according to the present application, the unlocking mechanism includes protrusions provided on an end face of the ejection member.

In some embodiments according to the present application, the protrusions are located between adjacent clamping member rib portions in the circumferential direction.

In some embodiments according to the present application, the cam body includes a large-diameter section and a small-diameter section, the ejection member being disposed between the small-diameter section and the clamping member.

In some embodiments according to the present application, the elastic clamping portion includes a plurality of fingers arranged at intervals in the circumferential direction, and the ejection member includes a plurality of legs arranged at intervals in the circumferential direction.

In some embodiments according to the present application, the biasing member is a spring.

In some embodiments according to the present application, the container manipulation device further includes a cap. The cap has an end fixed to the clamping member and a cylindrical portion extending from the end in an axial direction. The spring is accommodated between the cylindrical portion and the clamping member and is used to apply a force to the cam rib portion.

In another aspect of the present application, a medical detection analyzer is provided, including the above-described container manipulation device and a motor. The container manipulation device is operatively coupled to the motor and is capable of linear movement under the drive of the motor.

In some embodiments according to the present application, the medical detection analyzer is an immunoassay analyzer.

### Technical Effects

According to the container manipulation device and the medical detection analyzer of the present application, the pneumatic system in the conventional technology is replaced by an internal biasing member capable of storing and releasing energy, thereby significantly simplifying the structure, reducing costs, and/or improving reliability and stability.

The container manipulation device according to the present application includes a locking mechanism, so that the transmission of the force from the biasing member can be well controlled to prevent the container from being inadvertently released by subjected to unexpected force.

After the container is placed in place in a container disc, a reaction force from the container acts on the unlocking mechanism, thereby releasing the standby state of the ejection member. At this point, the biasing member applies a pushing force to the ejection member, pushing the ejection member and the container outward until the container is released from the clamping member. During the entire release process of the container, the container abuts against the container disc, so the release process of the container is smoother. In this way, the sloshing amplitude of the fluid (e.g., the reaction liquid) inside the container is significantly reduced, thereby effectively preventing the fluid inside the container from splashing onto external components (e.g., the ejection member or the clamping member) and avoiding the resulting contamination.

The effects of the present disclosure are not limited to the effects mentioned above, and those skilled in the art will clearly understand additional effects not described above from the description of the appended claims.

### BRIEF DESCRIPTION OF THE DRAWINGS

FIG. 1 is a schematic perspective view of a portion of a medical detection analyzer according to an embodiment of the present application;
FIG. 2 is a schematic view of the external of a container manipulation device of the medical detection analyzer of FIG. 1, in which the container manipulation device is clamping a container;
FIG. 3 is a schematic longitudinal cross-sectional view of the container manipulation device of FIG. 2 taken along line C-C;
FIG. 4 is a schematic view of the external of a clamping member of the container manipulation device of FIG. 2;
FIG. 5 is a schematic view of the external of a cam of the container manipulation device of FIG. 2;
FIG. 6 is a schematic view of the external of an ejection member of the container manipulation device of FIG. 2; and
FIGS. 7A to 7F are schematic views illustrating an operation process of the container manipulation device according to the present disclosure.

### DETAILED DESCRIPTION OF THE EMBODIMENTS

Hereinafter, various embodiments of the present disclosure will be described in detail with reference to the accompanying drawings, so that those skilled in the art can easily implement these embodiments. The following detailed description of the present disclosure is for illustrative purposes only and is in no way a limitation of the present disclosure, its application or usages. The embodiments described in this specification are not exhaustive and are merely some of multiple possible embodiments. The exemplary embodiments may be implemented in many different forms, and should not be construed as limiting the scope of the present disclosure. In some exemplary embodiments, well-known processes, well-known device structures, and well-known technologies may not be described in detail.

Portions irrelevant to the description will be omitted to clearly describe the present disclosure, and throughout the specification, the same reference numerals denote the same elements.

Furthermore, in the drawings, the dimensions and thicknesses of each element are arbitrarily illustrated for ease of description, and the present disclosure is not necessarily limited to those illustrated in the drawings. In the drawings, the thicknesses of layers, regions, or the like are exaggerated for clarity. In the drawings, the thicknesses of some layers and regions are exaggerated for ease of description.

In addition, it should be understood that when an element such as a layer, a film, a region, or a plate is referred to as being "on" or "above" another element, the element may be directly on the another element or an intermediate element may exist between the element and the another element. Conversely, when an element is referred to as being "directly on" another element, this means that no other intermediate elements exist between the two elements. Furthermore, the terms "on" or "above" mean being disposed on or below the referenced part, and do not necessarily mean being disposed on the upper end of the referenced part in the direction opposite to gravity.

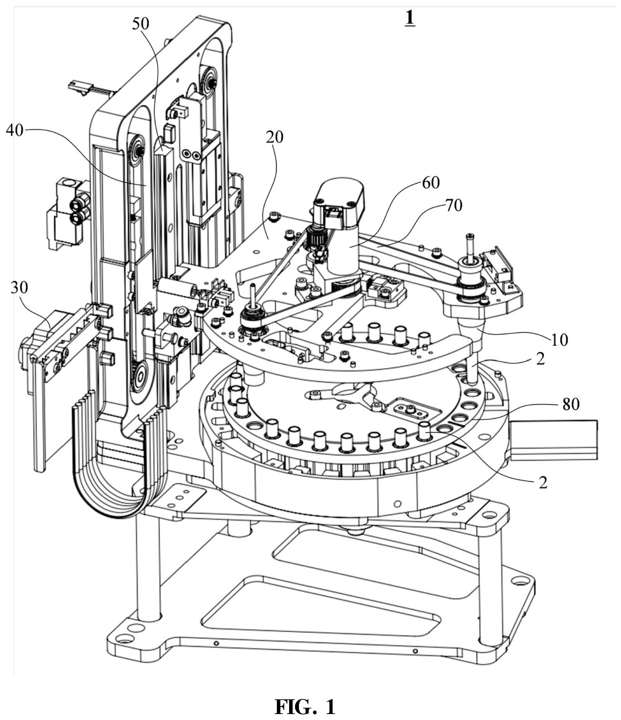

A medical detection analyzer according to the present application will be described below with reference to FIG. 1. It should be understood that the medical detection analyzer of the present application should not be limited to the specific example shown in FIG. 1, but may be modified as long as it is applicable to the concept of the present application. For example, the medical detection analyzer of the present application may be an immunoassay analyzer, such as a chemiluminescence immunoassay analyzer.

FIG. 1 is a schematic perspective view of a portion of a medical detection analyzer 1 according to an embodiment of the present application. As shown in FIG. 1, the medical detection analyzer 1 includes a container manipulation device 10 for manipulating a container 2, an arm 20 for supporting or mounting the container manipulation device 10, a motor 30 for moving the arm 20 up and down, a transmission mechanism 40 provided between the motor 30 and the arm 20, a guide mechanism 50 for guiding the up-and-down movement of the arm 20, a rotation motor 60 for rotating the container 2 clamped by the container manipulation device 10, a rotation transmission mechanism 70 provided between the rotation motor 60 and the container manipulation device 10, and a container disc 80 for holding the container 2.

The container manipulation device 10 is fixed to the arm 20. The structure of the arm 20 should not be limited in the present disclosure. For example, the arm 20 may have any suitable shape as needed. For example, the arm 20 may be used to support or mount one or more arms 20.

The motor 30, the transmission mechanism 40, and the guide mechanism 50 constitute a linear drive mechanism for moving the arm 20 up and down. In the illustrated example, the transmission mechanism 40 is a belt transmission mechanism, and the guide mechanism 50 is a guide mechanism in a form of a guide rail. However, it should be understood that the linear drive mechanism should not be limited to the specific example shown in the drawings, but may be modified as long as it can achieve the functions described herein.

The arm 20 moves up and down together with the container manipulation device 10 under the drive of the linear drive mechanism. As the container manipulation device 10 moves up and down, the container manipulation device 10 can clamp or release the container 2, which will be described in detail later.

The rotation motor 60 and the rotation transmission mechanism 70 constitute a rotary drive mechanism for rotating the container 2 clamped by the container manipulation device 10. In the illustrated example, the rotation transmission mechanism 70 is a belt transmission mechanism. However, it should be understood that the rotary drive mechanism should not be limited to the specific example shown in the drawings, but may be modified as long as it can achieve the functions described herein.

Under the drive of the rotary drive mechanism, the container clamped by the container manipulation device 10 rotates, thereby allowing the substances inside the container to be mixed. It should be understood that, depending on the operational requirements of the medical detection analyzer for the container, the rotary drive mechanism may be replaced with a drive mechanism for achieving other operational functions (for example, a drive mechanism for horizontal movement) or may be omitted.

The container disc 80 is configured to support or place the container 2. As shown in FIG. 1, the container disc 80 includes recesses or holes for holding the container 2. The container disc 80 may have an annular shape and may be provided with recesses or holes at predetermined intervals along the circumferential direction for holding the container 2. It should be understood that the structure of the container disc 80 should not be limited in the present disclosure, but may be modified as needed. For example, the structures of recesses or holes of the container disc 80 may be varied depending on the structure of the container 2.

The container 2 is used to hold reaction liquids, test reagents, analytical fluids, or the like, depending on the type of the medical detection analyzer. It should be understood that the structure of the container 2 should not be limited in the present application, but may have any suitable structure. For example, the container 2 may have an elongated tubular or cup-like shape.

For the medical detection analyzer 1 shown in FIG. 1, when in operation, the arm 20 and the container manipulation device 10 are moved downward by the motor 30, the transmission mechanism 40, and the guide mechanism 50. When the container manipulation device 10 contacts the container 2 on the container disc 80, the container manipulation device 10 is further driven downward, and a clamping member (to be described in detail later) of the container manipulation device 10 elastically opens and clamps the container 2 until the container 2 reaches a clamping position. The arm 20 and the container manipulation device 10 are moved in the opposite direction (i.e., upward) by the motor 30, the transmission mechanism 40, and the guide mechanism 50, clamping the container 2 and moving the container 2 upward away from the container disc 80.

After the container 2 leaves the container disc 80, the clamping member of the container manipulation device 10 and the container 2 are rotated by the rotation motor 60 and the rotation transmission mechanism 70. The container 2 may be rotated in a single direction or alternately in two opposite directions. The rotation causes the substances inside the container 2 to be mixed to promote, for example, a reaction.

Then, the drive of the rotation motor 60 is stopped, the motor 30 is started again, and the container manipulation device 10 and the clamped container 2 are moved downward. After the container 2 is placed in place in the container disc 80, an ejection member (to be described in detail later) of the container manipulation device 10 pushes or ejects the container 2 out. Then, the container manipulation device 10 moves upward away from the container disc 80 under the drive of the motor 30.

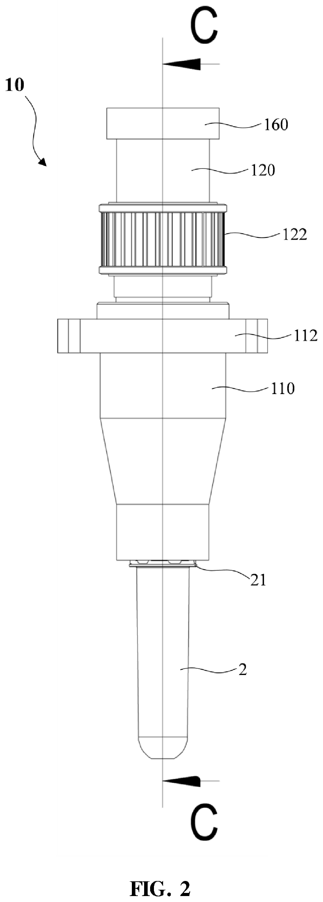

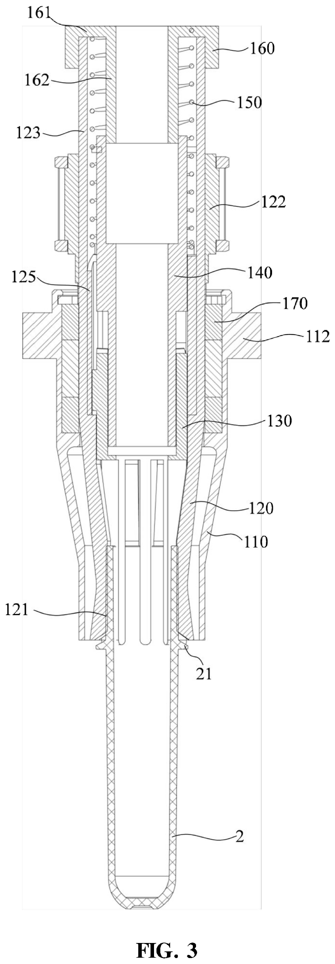

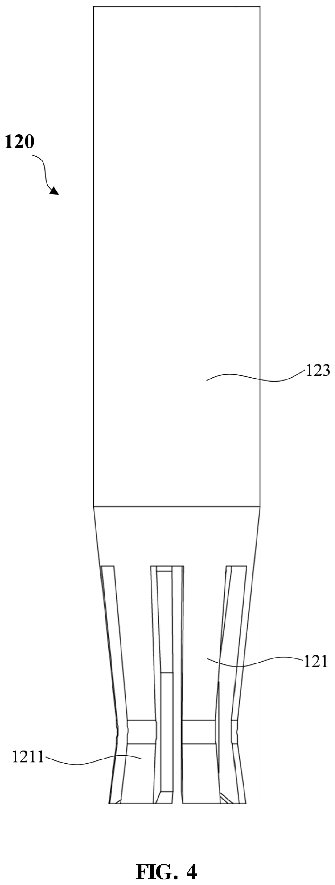

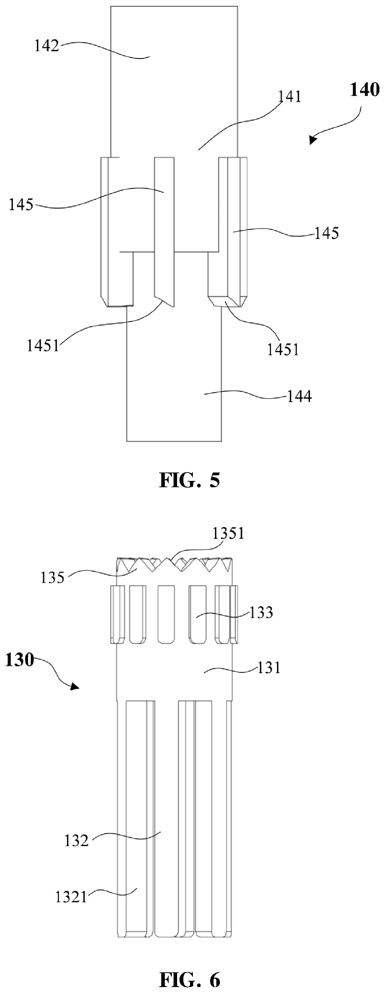

The container manipulation device 10 according to an embodiment of the present disclosure will be described below with reference to FIGS. 2 to 6. FIG. 2 is a schematic view of the external of the container manipulation device 10 of the medical detection analyzer of FIG. 1, in which the container manipulation device 10 is clamping a container 2; FIG. 3 is a schematic cross-sectional view of the container manipulation device 10 of FIG. 2 taken along a longitudinal section; FIG. 4 is a schematic view of the external of the clamping member 120 of the container manipulation device 10 of FIG. 2; FIG. 5 is a schematic view of the external of a cam 140 of the container manipulation device 10 of FIG. 2; and FIG. 6 is a schematic view of the external of the ejection member 130 of the container manipulation device 10 of FIG. 2.

Referring to FIGS. 2 and 3, the container manipulation device 10 includes a housing 110, the clamping member 120, the ejection member 130, the cam 140, a spring 150, and a cap 160.

The housing 110 is configured to be fixedly mounted or connected to the arm 20. The housing 110 includes a connecting portion 112. In the illustrated example, the housing 110 has a cylindrical shape similar to that of the clamping member 120. However, it should be understood that the structure of the housing 110 should not be limited to the specific example shown in the drawings, but may be modified as needed as long as it can achieve the functions described herein.

The clamping member 120 is configured to clamp the container 2. As shown, the container 2 may have a limiting portion 21 provided on its outer peripheral surface. Various positions of the container 2 can be determined by the limiting portion 21. For example, a clamping position where the container 2 is clamped in the clamping member 120, a placement or release position where the container 2 is placed in the container disc 80, or the like. When an end of the clamping member 120 abuts against the limiting portion 21, it indicates that the container 2 is in an appropriate or desired clamping position in the clamping member 120.

Referring to FIGS. 3 and 4, the clamping member 120 includes an elastic clamping portion 121 for clamping the container 2 and a cylindrical clamping member body 123. The elastic clamping portion 121 extends in an axial direction from one end of the clamping member body 123. The elastic clamping portion 121 may elastically open to clamp the container 2. In the example shown in the drawings, the elastic clamping portion 121 may include multiple fingers 1211 arranged at intervals in the circumferential direction. The multiple fingers 1211 may be spaced equidistantly in the circumferential direction to provide uniform clamping force on the container. The number of fingers is at least 2. It should be understood that the structure of the elastic clamping portion 121 should not be limited to the specific example shown in the drawings, but may be modified as long as it can achieve the functions described herein.

Referring to FIG. 3, a bearing 170 may be provided between the housing 110 and the clamping member body 123. The clamping member 120 may be coupled to the rotary drive mechanism (the rotation motor 60 and the rotation transmission mechanism 70) and, under the drive of the rotary drive mechanism, is rotatable relative to the housing 110. For this purpose, a driving portion 122, for example, a pulley driven by a belt, may be provided on the outer peripheral surface of the clamping member 120. It should be understood that when it is not necessary to rotate the container 2, the rotary drive mechanism and the driving portion may be omitted. In such a case, the housing 110 and the clamping member 120 may be integrally formed, or the housing 110 may be omitted and the clamping member 120 may be designed to be coupled to the linear drive mechanism.

The ejection member 130 is configured to push the container 2 out of the clamping member 120. The ejection member 130 is disposed in the clamping member 120 in a slidable manner. When it is necessary to place or release the container 2, the ejection member 130 is subjected to a force and moves downward relative to the clamping member 120, and transmits the force to the container 2 so as to push the container 2 out of the clamping member 120. In the example shown in the drawings, a pushing force for pushing the container 2 downward is applied to the ejection member 130 by the spring 150.

Referring to FIG. 6, the ejection member 130 includes a cylindrical ejection member body 131 and ejection member rib portions 133 provided on the outer peripheral surface of the ejection member body 131 and extending in the axial direction. The ejection member rib portions 133 each may be located between every two adjacent clamping member rib portions 125 in the circumferential direction. The ejection member rib portions 133 serve to guide the axial relative movement of the ejection member 130 relative to the clamping member 120, so that the ejection member 130 can slide axially relative to the clamping member 120 but cannot rotate.

The ejection member 130 may further include an elastic portion 132 extending in the axial direction from one end (the lower end in FIG. 6) of the ejection member body 131. The elastic portion 132 is configured to be elastically adaptable to the tapered elastic clamping portion 121 of the clamping member 120. In the example shown in the drawings, the elastic portion 132 includes multiple legs 1321 arranged at intervals in the circumferential direction. It should be understood that the structure of the elastic portion 132 should not be limited to the specific example shown in the drawings, but may be modified as long as it can achieve the functions described herein.

In order to reliably control the clamping and releasing operations on the container 2, the cam 140 is provided. The cam 140 may be disposed between the spring 150 and the ejection member 130. The cam 140 may be configured to be engaged with the clamping member 120 or the ejection member 130 to control the transmission of the force from the spring 150 or to control the relative movement between the ejection member 130 and the clamping member 120.

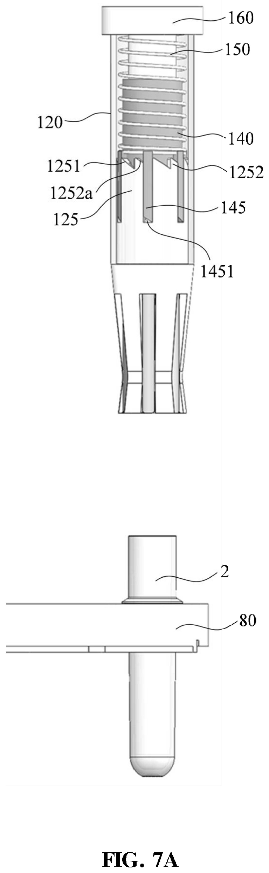

The structure of the engagement of the cam 140 with the clamping member 120 or the ejection member 130 will be described below with reference to FIGS. 7A to 7F. In order to clearly understand the internal structure of the container manipulation device 10, FIGS. 7A to 7F are drawn in a see-through manner.

Referring to FIGS. 7A to 7F, the clamping member 120 may include clamping member rib portions 125 provided on the inner peripheral surface of the clamping member body 123. The clamping member rib portions 125 protrude radially inward from the inner peripheral surface of the clamping member body 123. Correspondingly, referring to FIG. 5 and FIGS. 7A to 7F, the cam 140 includes a cylindrical cam body 141 and cam rib portions 145 provided on the outer peripheral surface of the cam body 141.

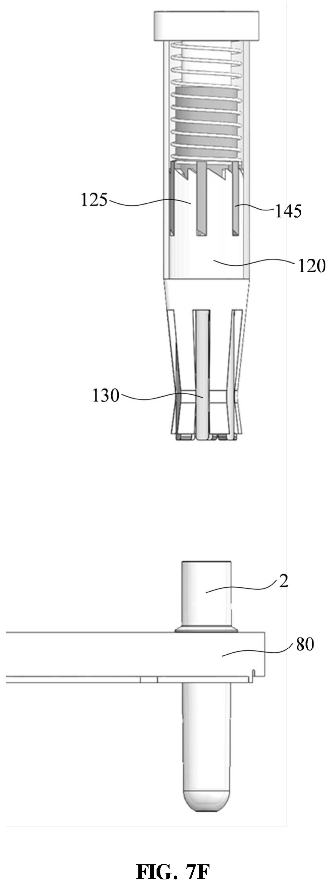

The clamping member rib portions 125 may extend in the axial direction. Correspondingly, the cam rib portions 145 may extend in the axial direction. When the cam 140 is in the position shown in FIGS. 7A and 7F, the cam rib portions 145 each are located between every two adjacent clamping member rib portions 125. In this way, the axial movement of the cam 140 relative to the clamping member 120 can be guided.

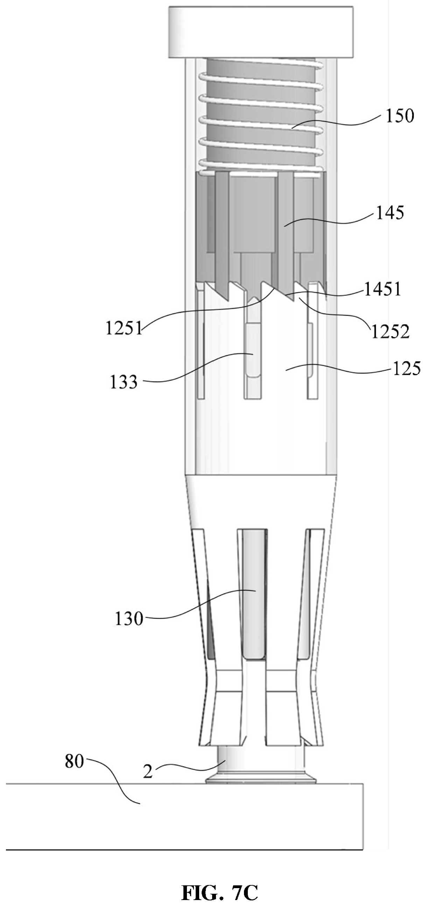

An inclined surface 1251 and a stop portion 1252 may be provided on the top surface (also referred to as a free end surface) of each clamping member rib portion 125 (see FIGS. 7A to 7F). The cam rib portions 145 extend radially outward from the outer peripheral surface of the cam body 141. Each cam rib portion 145 may be provided with an inclined surface 1451. Referring to FIG. 7C, the inclined surface 1451 on each cam rib portion 145 may slide on the inclined surface 1251 of each clamping member rib portion 125 and be stopped by the stop portion 1252 of each clamping member rib portion 125. In the illustrated example, the inclined surface 1451 of each cam rib portion 145 constitutes a first engagement portion, and the inclined surface 1251 and the stop portion 1252 of each clamping member rib portion 125 constitute a second engagement portion.

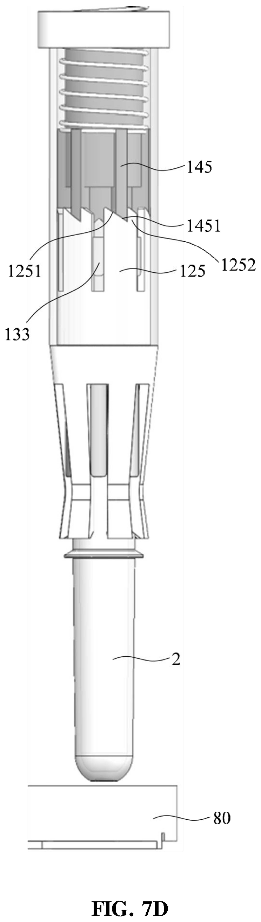

When the first engagement portion of the cam 140 is engaged with the second engagement portion of the clamping member 120 as shown in FIGS. 7C and 7D, the axial position of the cam 140 relative to the clamping member 120 is fixed, and at this time, the elastic force of the spring 150 is counteracted by the clamping member 120. In this way, the ejection member 130 is in a standby state in which the force applied by the spring 150 will not be transmitted to the container 2. During the process of lifting the container 2 shown in FIGS. 7C to 7D, the container 2 will not be inadvertently subjected to a force and released from the clamping member 120. Therefore, the first engagement portion of the cam 140 and the second engagement portion of the clamping member 120 constitute a locking mechanism described in the present disclosure.

The locking mechanism according to the present disclosure is configured such that when the container 2 is clamped in place by the clamping member 120 (specifically, the elastic clamping portion 121), the ejection member 130 may be placed in the standby state in which the force applied by the spring 150 will not be transmitted to the container 2. It should be understood that the locking mechanism should not be limited to the specific example shown in the drawings, but may be modified as long as it can achieve the functions described herein.

An end of the ejection member body 131 opposite to the elastic portion 132 (the upper end in FIG. 6) may be configured to be engaged with the cam 140, to drive the cam 140, or to be operable in cooperation with the cam 140.

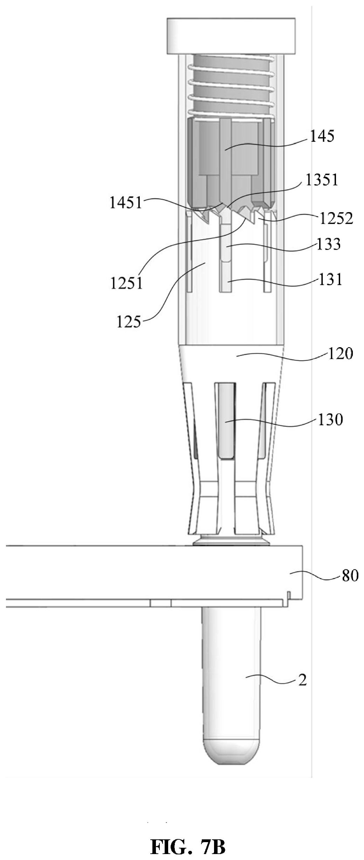

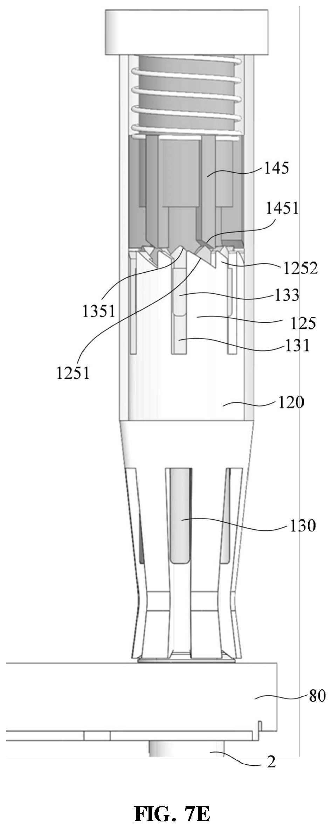

Referring to FIG. 6, FIG. 7B, and FIG. 7E, protrusions 135 are provided on the upper end face of the ejection member body 131. The protrusions 135 each may be located between every two adjacent clamping member rib portions 125 in the circumferential direction. The protrusions 135 may be configured to push the cam rib portions 145 when in the position shown in FIG. 7B, so that the inclined surfaces 1451 of the cam rib portions 145 can slide onto the inclined surfaces 1251 of the clamping member rib portions 125. The protrusions 135 may be configured to lift the cam rib portions 145 when in the position shown in FIG. 7E, so that the cam rib portions 145 can pass over the stop portions 1252. When the cam rib portions 145 pass over the stop portions 1252 and enter between adjacent clamping member rib portions 125, the cam 140 can slide axially relative to the clamping member 120. At this time, the elastic force of the spring 150 is applied to the cam 140, the cam 140 transmits the force to the ejection member 130, and the ejection member 130 in turn transmits the force to the container 2, thereby pushing the container 2 out. Therefore, the protrusions 135 constitute an unlocking mechanism described in the present disclosure.

The unlocking mechanism according to the present disclosure is configured to release the standby state of the ejection member 130 so that the container 2 is pushed out from the clamping member 120 under the action of the spring 150. It should be understood that the unlocking mechanism according to the present disclosure should not be limited to the specific example shown in the drawings, but may be modified as long as it can achieve the functions described herein.

Each of the protrusions 135 of the ejection member 130 may have an inclined surface 1351. As shown in FIG. 6, the upper end face of the cam 140 may be configured in a serrated shape. In addition, each stop portion 1252 of the clamping member 120 may have an inclined surface (i.e., a top surface) 1252a. In this way, under the interaction of the inclined surfaces 1451 of the cam 140 with the inclined surfaces 1351 of the ejection member 130, as well as the inclined surfaces 1251 and 1252a of the clamping member 120, the cam 140 is enabled to move in the circumferential direction. That is, the cam 140 is rotatable between a first circumferential position shown in FIGS. 7A, 7B, and 7F and a second circumferential position shown in FIGS. 7C to 7E.

When the cam 140 is in the first circumferential position, each of the clamping member rib portions 125 is located between every two adjacent cam rib portions 145 in the circumferential direction. At this time, relative sliding between the clamping member 120 and the cam 140 can be achieved.

When the cam 140 is in the second circumferential position, the cam rib portions 145 are engaged with the clamping member rib portions 125, so that the clamping member 120 can resist the elastic force of the spring 150, thereby placing the ejection member 130 in the standby state in which no pushing force is applied to the container 2.

Referring again to FIG. 5, the cam body 141 may include a large-diameter section 142 and a small-diameter section 144. The ejection member 130 may be disposed between the small-diameter section 144 and the clamping member 120. The cam rib portions 145 may extend radially outward from outer peripheral surfaces of both the large-diameter section 142 and the small-diameter section 144. Such a structure is more compact.

Referring back to FIG. 3, the cap 160 may have an end portion 161 fixed to the clamping member 120 and a cylindrical portion 162 extending in the axial direction from the end portion 161. The spring 150 is accommodated between the cylindrical portion 162 and the clamping member 120. The lower end of the spring 150 abuts against upper end faces of the cam rib portions 145 to apply a force to the cam 140.

The spring 150 constitutes a biasing member described in the present disclosure. The biasing member according to the present disclosure is configured to apply a force to the ejection member 130 so that the ejection member 130 can push the container 2 out from the elastic clamping portion 121 of the clamping member 120. It should be understood that the biasing member according to the present disclosure should not be limited to the specific example shown in the drawings, but may be modified as long as it can achieve the functions described herein.

The container manipulation device 10 according to the present disclosure can achieve the clamping (or picking up) and releasing (or pushing out) operations of the container under the drive of a single motor 30 alone. There is no need to additionally provide a pneumatic system. Therefore, the structure of the container manipulation device and the medical detection analyzer can be significantly simplified, the cost of the container manipulation device and the medical detection analyzer can be greatly reduced, and the reliability and stability of the container manipulation device and the medical detection analyzer can be improved.

The operation process of the container manipulation device 10 according to the present disclosure will be described below with reference to FIGS. 7A to 7F.

FIG. 7A shows a state in which the container manipulation device 10 is ready to pick up a container 2 placed in the container disc 80. As shown in FIG. 7A, the cam rib portions 145 of the cam 140 each are located between every adjacent clamping member rib portions 125. Under the action of the spring 150, the cam 140 and the ejection member 130 are in a lower position relative to the clamping member 120. At this time, the lower end of the ejection member 130 is substantially flush with the lower end of the clamping member 120.

FIG. 7B shows a state in which the container manipulation device 10 is picking up the container 2, where the container 2 has reached the clamping position. As shown in FIG. 7B, the container manipulation device 10 moves downward, and the elastic clamping portion 121 of the clamping member 120 expands to receive the container 2. As the container manipulation device 10 moves further downward, the container 2 pushes the ejection member 130 and the cam 140 to move upward relative to the clamping member 120. When the container 2 reaches the desired clamping position, the protrusions 135 of the ejection member 130 lift the cam rib portions 145 so that they are higher than the clamping member rib portions 125.

Under the interaction of the inclined surfaces 1351 of the protrusions 135 and the inclined surfaces 1451 of the cam rib portions 145, the cam rib portions 145 are enabled to slide onto the inclined surfaces of the clamping member rib portions 125 of the clamping member 120 and are stopped by the stop portions 1252, as shown in FIG. 7C.

Since the cam 140 is engaged with the clamping member 120, the clamping member 120 can resist the elastic force of the spring 150, so the elastic force of the spring 150 will not be transmitted to the ejection member 130 and the container 2. In this way, the container manipulation device 10 can be raised, and the container 2 also rises away from the container disc 80 while being clamped by the clamping member 120, as shown in FIG. 7D.

When it is necessary to place or release the container 2 into the container disc 80, the container manipulation device 10 is lowered until the container 2 is completely seated in the recess or hole of the container disc 80. At this time, as shown in FIG. 7E, the protrusions 135 of the ejection member 130 lifts the cam rib portions 145 so that they are higher than the stop portions 1252 of the clamping member 120. Under the interaction of the inclined surfaces 1451 of the cam rib portions 145 with both the inclined surfaces 1351 of the protrusions 135 and the inclined surfaces 1252a of the stop portions 1252, the cam rib portions 145 each slide between every adjacent clamping member rib portions 125.

At this time, under the action of the spring 150, the cam 140 and the ejection member 130 move downward and push the container 2 downward until the container 2 is completely pushed out of the clamping member 120. Then, the container manipulation device 10 is raised to the initial position by the motor 30, as shown in FIG. 7F.

Although the present disclosure has been described with reference to exemplary embodiments, it should be understood that the present disclosure is not limited to the specific embodiments described and shown in detail herein. Without departing from the scope defined by the claims, those skilled in the art can make various changes to the exemplary embodiments. The features in the various embodiments may be combined with each other where no conflict exists. Alternatively, a feature in an embodiment may also be omitted.

## Claims

1. A container manipulation device for a medical detection analyzer, comprising:
a clamping member comprising an elastic clamping portion for clamping a container;
an ejection member slidably disposed in the clamping member;
a biasing member configured to apply a force to the ejection member such that the ejection member pushes the container out of the elastic clamping portion;
a locking mechanism configured to place the ejection member in a standby state where the force applied by the biasing member will not be transmitted to the container when the container is clamped in place by the elastic clamping portion; and
an unlocking mechanism configured to release the standby state of the ejection member, thereby pushing the container out of the clamping member under the action of the biasing member.

2. The container manipulation device according to claim 1, further comprising a housing, wherein a bearing is disposed between the housing and the clamping member,
the housing is configured to be coupled to a linear drive mechanism and to move linearly together with the clamping member under the drive of the linear drive mechanism,
and the clamping member is configured to be coupled to a rotational drive mechanism and to be rotatable relative to the housing under the drive of the rotational drive mechanism.

3. The container manipulation device according to claim 1 or 2, further comprising a cam disposed between the biasing member and the ejection member, wherein
the locking mechanism comprises a first engagement portion provided on the cam and a second engagement portion provided on the clamping member, the first engagement portion being configured to be engaged with the second engagement portion to place the ejection member in the standby state; and
the unlocking mechanism is provided on the ejection member.

4. The container manipulation device according to claim 3, wherein the cam comprises a cylindrical cam body and cam rib portions disposed on an outer peripheral surface of the cam body, the first engagement portion being provided on the cam rib portions, and
the clamping member comprises a cylindrical clamping member body and clamping member rib portions disposed on an inner peripheral surface of the clamping member body, the second engagement portion being provided on the clamping member rib portions.

5. The container manipulation device according to claim 4, wherein the cam is configured to be rotatable between a first circumferential position and a second circumferential position,
when the cam is at the first circumferential position, each of the clamping member rib portions is located between adjacent cam rib portions in a circumferential direction so as to enable relative axial sliding between the clamping member and the cam;
when the cam is at the second circumferential position, the first engagement portion is engaged with the second engagement portion.

6. The container manipulation device according to claim 5, wherein the clamping member rib portions extend in an axial direction; and/or
the cam rib portions extend in the axial direction.

7. The container manipulation device according to claim 5, wherein the first engagement portion is provided on an end portion of the cam rib portions;
and the second engagement portion is provided on an end portion of the clamping member rib portions.

8. The container manipulation device according to claim 7, wherein each of the first engagement portion, the second engagement portion, and the unlocking mechanism is provided with an inclined surface,
and the container manipulation device is configured such that: under the interaction between the inclined surface of the first engagement portion and the inclined surface of the second engagement portion, and under the interaction between the inclined surface of the first engagement portion and the inclined surface of the unlocking mechanism, the cam is enabled to rotate between the first circumferential position and the second circumferential position.

9. The container manipulation device according to claim 8, wherein the second engagement portion has a stop portion configured to allow the first engagement portion to rest against the clamping member.

10. The container manipulation device according to any one of claims 4 to 9, wherein the ejection member comprises a cylindrical ejection member body and ejection member rib portions disposed on an outer peripheral surface of the ejection member body,
the ejection member rib portions is located between adjacent clamping member rib portions in a circumferential direction such that the ejection member is axially slidable but non-rotatable relative to the clamping member,
and the unlocking mechanism is provided on an end surface of the ejection member body facing the cam.

11. The container manipulation device according to claim 10, wherein the ejection member rib portions extend in an axial direction.

12. The container manipulation device according to claim 10, wherein the unlocking mechanism comprises protrusions provided on the end surface of the ejection member.

13. The container manipulation device according to claim 12, wherein the protrusions are located between adjacent clamping member rib portions in a circumferential direction.

14. The container manipulation device according to any one of claims 4 to 9, wherein the cam body comprises a large-diameter section and a small-diameter section,
the ejection member being disposed between the small-diameter section and the clamping member.

15. The container manipulation device according to claim 1 or 2, wherein the elastic clamping portion comprises a plurality of fingers disposed at intervals in a circumferential direction; and
the ejection member comprises a plurality of legs disposed at intervals in a circumferential direction.

16. The container manipulation device according to claim 1 or 2, wherein the biasing member is a spring.

17. The container manipulation device according to claim 16, further comprising a cap having an end fixed to the clamping member and a cylindrical portion extending in an axial direction from the end,
wherein the spring is accommodated between the cylindrical portion and the clamping member, and is configured to apply a force to the cam rib portions.

18. A medical detection analyzer, comprising the container manipulation device according to any one of claims 1 to 17 and a motor, wherein
the container manipulation device is operatively coupled to the motor and is capable of linear movement under the drive of the motor.

19. The medical detection analyzer according to claim 18, wherein the medical detection analyzer is an immunoassay analyzer.
